## Europäisches Patentamt
## European Patent Office
## Office européen des brevets

(19)

(11) Numéro de publication : **0 018 247**
**B1**

(12)

# FASCICULE DE BREVET EUROPÉEN

(45) Date de publication du fascicule du brevet :
23.05.84

(51) Int. Cl.³ : **G 21 K   1/08**, **H 01 J 49/20**

(21) Numéro de dépôt : **80400389.5**

(22) Date de dépôt : **21.03.80**

(54) **Dispositif de déviation magnétique stigmatique et achromatique d'un faisceau de particules chargées et appareil d'irradiation utilisant un tel dispositif.**

(30) Priorité : 03.04.79 FR 7908370

(43) Date de publication de la demande :
29.10.80 Bulletin 80/22

(45) Mention de la délivrance du brevet :
23.05.84 Bulletin 84/21

(84) Etats contractants désignés :
DE GB NL SE

(56) Documents cités :
FR-A- 2 058 485
FR-A- 2 215 011
NUCLEAR INSTRUMENTS AND METHODS vol. 80, no.
2, avril 1970, Amsterdam, NL J. AUCOUTURIER et al.
"Systèmes déviateurs de faisceaux stigmatiques et
achromatiques", pages 268-276

(73) Titulaire : **C.G.R. MeV**
**Route de Guyancourt**
**F-78530 Buc (FR)**

(72) Inventeur : **Tronc, Dominique**
**"THOMSON-CSF" SCPI - 173, Bld Haussmann**
**F-75360 Paris Cedex 08 (FR)**

(74) Mandataire : **Grynwald, Albert et al**
**THOMSON-CSF SCPI 173, Bld Haussmann**
**F-75379 Paris Cedex 08 (FR)**

## Description

La présente invention a pour objet un dispositif de déviation magnétique achromatique permettant de dévier d'un angle $\phi$ un faisceau de particules chargées, accélérées (des électrons par exemple), ces particules pouvant présenter un large domaine de moments de quantité de mouvement.

Le dispositif de déviation suivant l'invention permet en particulier, de dévier d'un angle $\phi > \pi$ un faisceau d'électrons accélérés entre 10 et 20 MeV par exemple, sans avoir à modifier les valeurs des champs magnétiques créés dans les entrefers des pièces polaires équipant le dispositif de déviation.

Suivant l'invention, un dispositif de déviation magnétique stigmatique, achromatique d'un faisceau de particules chargées, accélérées, comprenant au moins un électroaimant muni de pièces polaires délimitant des entrefers dans lesquels sont créés des champs magnétiques de même sens et de valeurs déterminées de façon que les trajectoires des particules aient la forme de boucles dont les longueurs sont fonction du moment de quantité de mouvement de ces particules, l'ensemble de ces secteurs magnétiques ayant un plan de symétrie perpendiculaire de la trajectoire moyenne du faisceau de particules et coupant ce plan suivant un axe XX de symétrie du dispositif de déviation, caractérisé en ce qu'il comporte un premier, un second et un troisième secteurs magnétiques accolés, en ce que ces secteurs présentent successivement au faisceau de particules une face d'entrée E plane, une première face galbée $F_1$ commune aux premier et second secteurs accolés, une seconde face galbée $F_2$ commune aux second et troisième secteurs accolés et une face de sortie S plane, les faces d'entrée E et de sortie S faisant entre elles un angle $2\,\alpha$, en ce que les différentes trajectoires des particules sont orthogonales aux faces galbées $F_1$ et $F_2$ ainsi qu'à l'axe XX de symétrie, et en ce que les valeurs des inductions magnétiques créées dans les premier et troisième secteurs magnétiques sont égales à $KB_0$, $B_0$ étant la valeur de l'induction magnétique dans le second secteur magnétique et K un coefficient numérique inférieur à 1.

L'invention sera mieux comprise et d'autres caractéristiques apparaîtront à l'aide de la description ci-après et des dessins qui l'accompagnent et sur lesquels :

la figure 1    représente un premier exemple de réalisation d'un dispositif de déviation magnétique suivant l'invention ;

la figure 2    représente les trajectoires de particules dans l'exemple de réalisation de la figure 1 ;

la figure 3    montre un deuxième exemple de réalisation d'un dispositif de déviation magnétique suivant l'invention ;

la figure 4    montre les trajectoires des particules dans le dispositif de la figure 3 ;

les figures 5 et 6    représentent respectivement la variation du rapport K des rayons de courbure des différentes trajectoires pour les exemples de réalisation montrés en figures 1 et 3 ;

les figures 7 et 8    montrent respectivement une vue de dessus et une coupe, suivant l'axe XX de symétrie, d'une paire de pièces polaires utilisées dans le dispositif suivant l'invention ;

la figure 9    représente l'effet de lentille obtenu dans le plan horizontal, avec les dispositifs des figures 2 et 3 ;

les figures 10 et 11    représentent respectivement une variante du dispositif suivant l'invention et les effets de lentille de ce dispositif sur le faisceau, dans les plans horizontal et vertical.

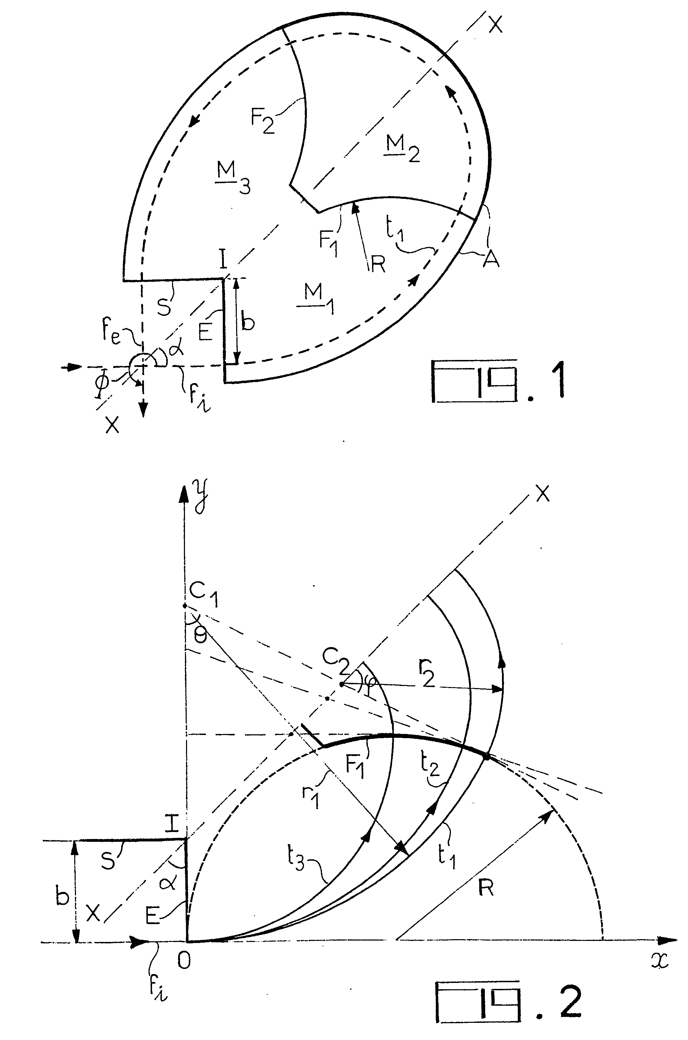

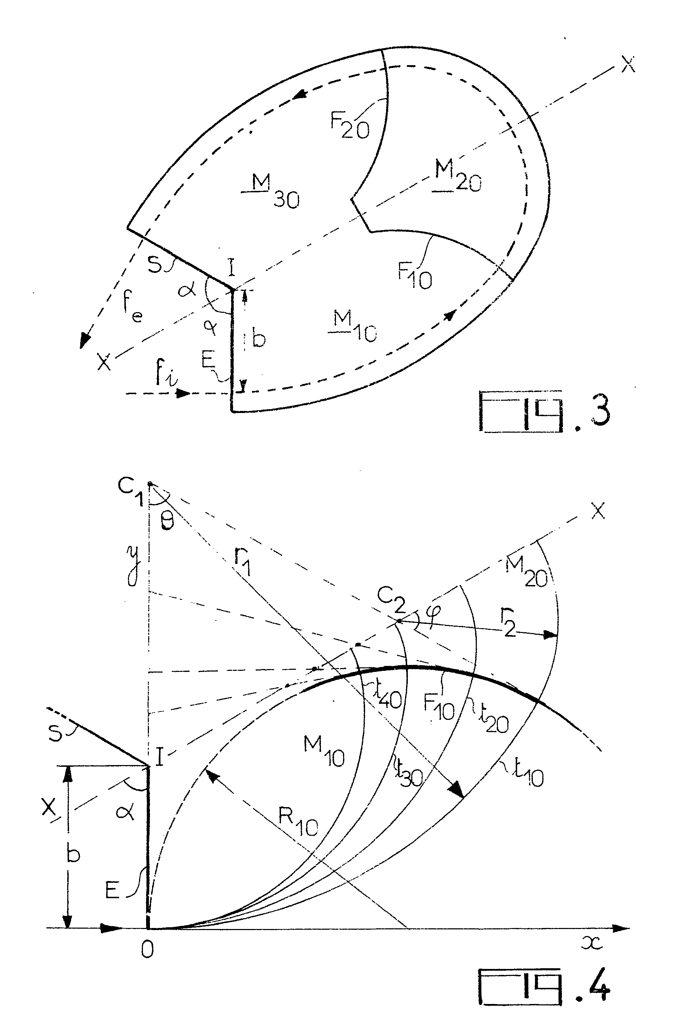

Le dispositif de déviation magnétique achromatique suivant l'invention, tel que montré en figure 1, permettant de dévier de 270° un faisceau de particules chargées, des électrons notamment, est constitué d'un électro-aimant comportant des bobinages magnétiques (non visibles sur la figure) et muni d'une paire de pièces polaires A, A (seule une pièce polaire A est visible sur la figure) de forme telle qu'elles délimitent trois secteurs magnétiques $M_1$, $M_2$, $M_3$, ayant un plan de symétrie perpendiculaire au plan dans lequel se déplace les trajectoires moyennes du faisceau de particules et coupant ce plan suivant un axe XX incliné d'un angle $\alpha = \pi/4$ par rapport à la trajectoire moyenne du faisceau $f_i$ incident. Le secteur magnétique $M_1$ est délimité par une face d'entrée E plane et une face $F_1$ de forme sensiblement circulaire, de rayon de courbure R, le secteur magnétique $M_3$ est délimité par une face de sortie S plane et une face $F_2$ identique à la face $F_1$ et le secteur magnétique $M_2$ intermédiaire accolé étant délimité par les faces $F_1$ et $F_2$. Les faces d'entrée E et de sortie S font entre elles un angle $2\,\alpha = \pi/2$.

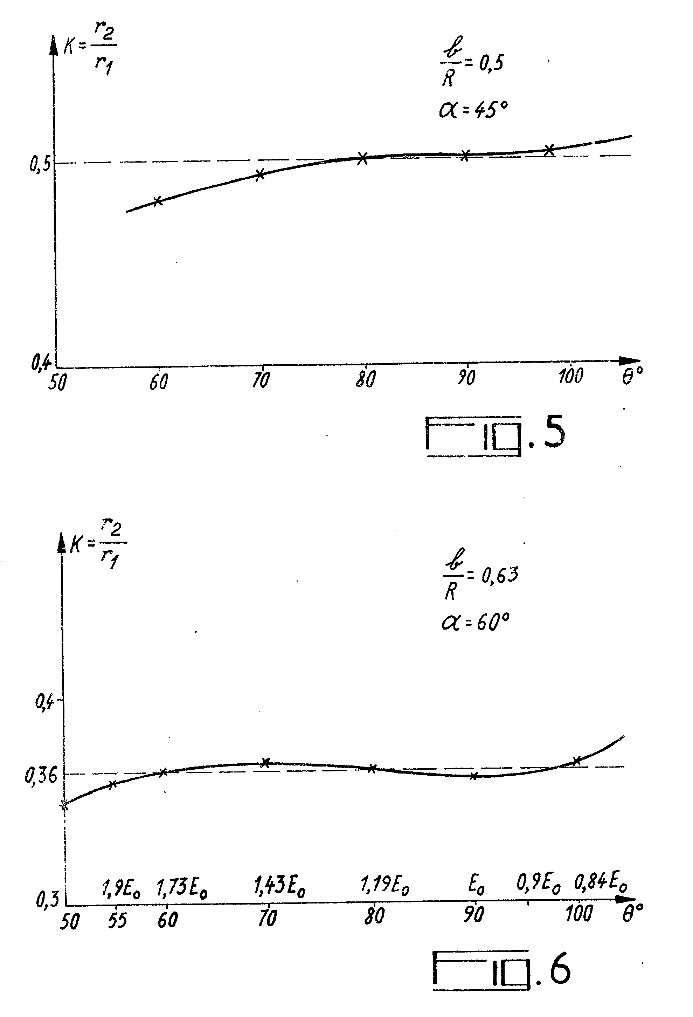

Les hauteurs des entrefers des secteurs magnétiques $M_1$ et $M_3$ d'une part, et $M_2$ d'autre part, sont telles que les valeurs des inductions magnétiques créées respectivement dans ces secteurs magnétiques $M_1$, $M_2$, $M_3$, sont égales à $B_0/2$, $B_0$, $B_0/2$, les particules étant alors déviées d'un angle $\theta$ dans chacun des secteurs magnétiques $M_1$ et $M_3$ et d'un angle $2\,\varphi$ dans le secteur magnétique $M_2$, la somme $2\,\theta + 2\,\varphi$ de ces angles étant égale à $2\,\pi - 2\,\alpha = 3\,\pi/2$ (figure 2).

La figure 2 montre les trajectoires $t_1$, $t_2$ et $t_3$ des particules ayant respectivement une énergie $E_1$, $E_2$, $E_3$. La trajectoire $t_1$ a un centre de courbure $C_1$ dans le secteur magnétique $M_1$ et un centre de courbure $C_2$ dans le secteur magnétique $M_2$. Cette trajectoire $t_1$ est orthogonale d'une part aux faces $F_1$, $F_2$ et, d'autre part à l'axe XX de symétrie du dispositif. Dans le dispositif de déviation magnétique suivant l'invention, le centre de courbure $C_2$ des trajectoires dans le secteur magnétique $M_2$ doit être situé sur l'axe XX de symétrie du dispositif de déviation. Ce centre de courbure $C_2$ des trajectoires peut être défini, dans un plan orthonormé xy, tel que montré sur la figure 2, par les relations :

2

$$x_{C_2} = (r_1 - r_2) \sin \theta \tag{1}$$

$$y_{C_2} = r_1(1 - \cos \theta) + r_2 \cos \theta \tag{2}$$

$r_1$ étant le rayon de courbure des trajectoires dans le secteur magnétique $M_1$ (et dans le secteur magnétique $M_3$ non représenté) et $r_2$ étant le rayon de courbure des trajectoires dans le secteur magnétique $M_2$.

Pour que les centres de courbure $C_2$ soient situés sur l'axe de symétrie XX, il faut que soit vérifiée la relation :

$$r_1(1 - \cos \theta) + r_2 \cos \theta = \frac{1}{\text{tg } \alpha} (r_1 - r_2) \sin \theta + b . \tag{3}$$

Si l'on pose : $r_2/r_1 = K$, la relation (3) devient

$$1 - \cos \theta + K \cos \theta = \frac{1}{\text{tg } \alpha} (1 - K) \sin \theta + \frac{b}{r_1} \tag{4}$$

$$\text{mais : } r_1 = \frac{R}{\text{tg } \frac{\theta}{2}}, \text{ donc } \frac{b}{r_1} = \frac{b}{R} \text{tg } \frac{\theta}{2}$$

R étant le rayon de courbure des faces $F_1$, $F_2$.

La valeur de K est alors donnée par la relation :

$$K = 1 + \frac{\left(\frac{b}{R} \text{tg } \frac{\theta}{2} - 1\right) \text{tg } \alpha}{\cos \theta \text{ tg } \alpha + \sin \theta} .$$

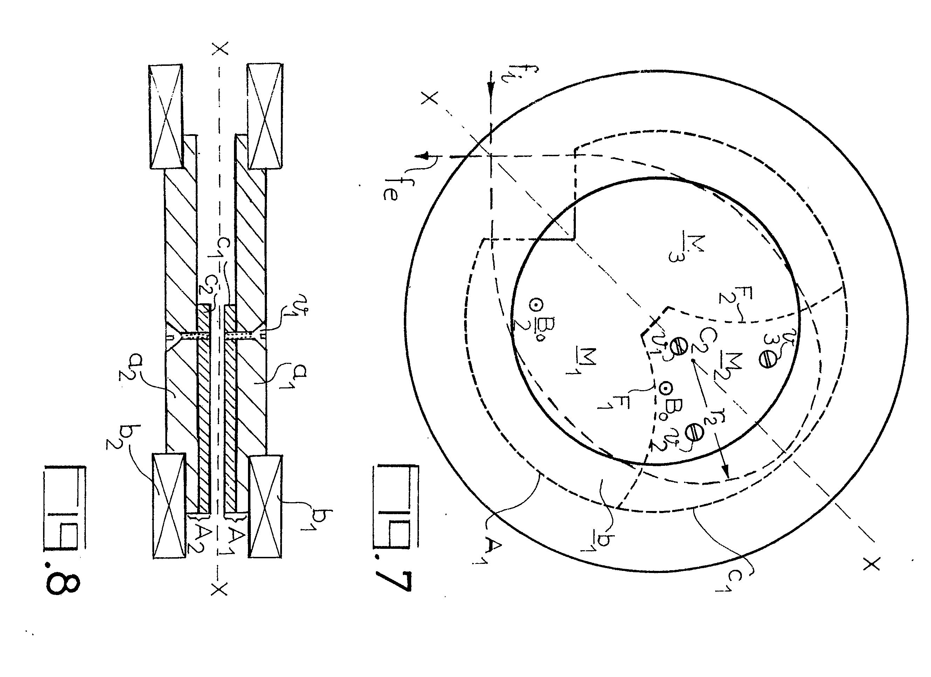

La figure 5 montre la variation de K en fonction de $\theta$, pour $\alpha = 45°$, $b/R = 0,5$.

Il est à noter que K est sensiblement égal à 0,5 pour des valeurs de $\theta$ comprises entre 75° et 100°, ce qui correspond à un domaine d'énergie compris entre 1,4 $E_0$ et 0,8 $E_0$.

La figure 3 représente un autre exemple de réalisation d'un dispositif de déviation magnétique suivant l'invention permettant de dévier le faisceau $f_i$ incident d'un angle 2 $\alpha$ égal à 240°, cette déviation étant achromatique. Ce dispositif de déviation magnétique comprend un électro-aimant comportant des bobinages magnétiques (non représentés) et muni d'une paire de pièces polaires de forme et de dimensions telles qu'elle délimite trois secteurs magnétiques $M_{10}$, $M_{20}$, $M_{30}$ accolés. Le secteur magnétique $M_{10}$ présente au faisceau une face d'entrée E plane et une face $F_{10}$ ayant la forme d'un arc de cercle de rayon $R_{10}$, le secteur magnétique $M_{30}$ comporte une face S de sortie plane et une face $F_{20}$ identique à la face $F_{10}$, tandis que le secteur magnétique $M_{20}$ accolé aux secteurs magnétiques $M_{10}$, $M_{30}$ est délimité par les faces $F_{10}$ et $F_{20}$. Les hauteurs des entrefers des secteurs magnétiques $M_{10}$, $M_{20}$ et $M_{30}$ sont telles que les inductions magnétiques créées dans chacun de ces secteurs sont égales respectivement à $KB_0$, $B_0$ et $KB_0$.

La figure 4 montre de façon détaillée les différentes trajectoires des particules de différentes énergies dans le dispositif de déviation montré en figure 3. Dans ce cas de réalisation, le rapport b/R a été choisi égal à 0,63, b étant la distance séparant la trajectoire moyenne du faisceau $f_i$ incident du point I, intersection de l'axe XX avec la face E d'entrée du dispositif de déviation. Pour les différentes trajectoires $t_{10}$, $t_{20}$, $t_{30}$, $t_{40}$ représentées, les centres de courbure $C_2$ dans le secteur magnétique $M_{20}$ sont sensiblement placés sur l'axe XX de symétrie. Ces différentes trajectoires $t_{10}$, $t_{20}$... correspondent à des particules d'énergie respectivement égales à $E_{10}$, $E_{20}$, $E_{30}$, $E_{40}$.

La figure 6 représente les variations de $K = r_2/r_1$ en fonction de $\theta$. On peut noter que, dans cet exemple de réalisation (figure 3) K est sensiblement égal à 0,36 pour des valeurs de $\theta$ comprises entre 55° et 100° et les inductions magnétiques créées dans les entrefers des secteurs magnétiques $M_{10}$, $M_{20}$ et $M_{30}$ sont respectivement égales à 0,36 $B_0$, $B_0$ et 0,36 $B_0$.

Dans les exemples de réalisation montrés en figures 1 et 3, les différences de valeurs des inductions magnétiques dans les secteurs $M_1$, $M_3$ et le secteur $M_2$ ont été obtenues avec différentes hauteurs des entrefers de ces secteurs magnétiques $M_1$, $M_3$ et $M_2$.

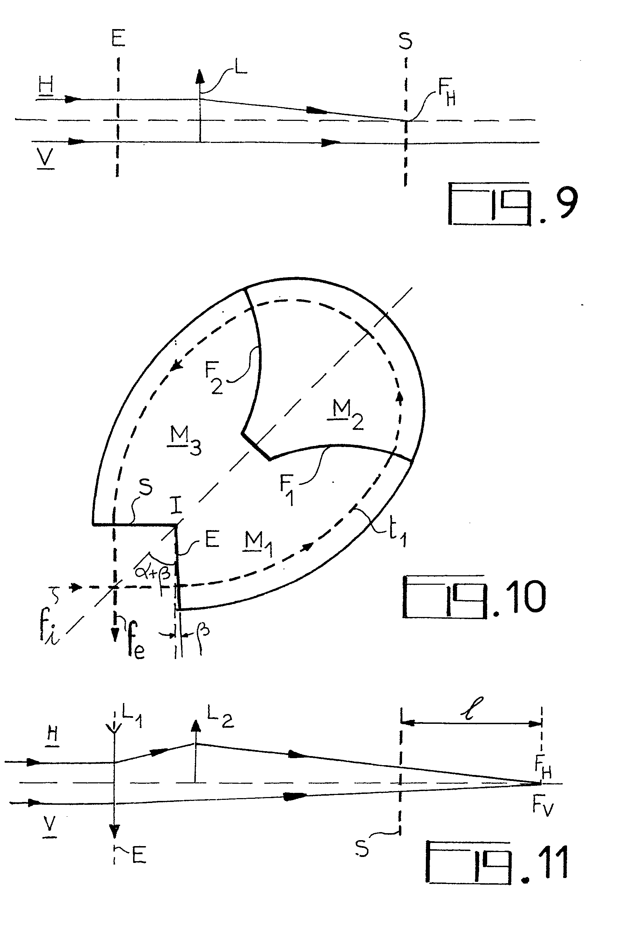

En figure 7, est montré un exemple de réalisation d'une pièce polaire $A_1$ suivant l'invention et le bobinage magnétique qui lui est associé. La pièce polaire $A_1$, de forme circulaire, est constituée d'un élément $a_1$ (figure 8) en matériau magnétique, du fer doux par exemple, dont les dimensions sont définies par les caractéristiques de fonctionnement du dispositif de déviations (type des particules, énergie de ces dernières, valeur des inductions magnétiques utilisées), et d'un élément $c_1$ superposé à l'élément $a_1$ et

fixé sur ce dernier au moyen de trois vis $v_1$, $v_2$, $v_3$ par exemple, cet élément $c_1$ délimitant le secteur magnétique intermédiaire $M_2$ (ou $M_{20}$). Les épaisseurs des éléments $a_1$ et $c_1$ sont choisies suivant la valeur des inductions magnétiques utilisées dans les secteurs magnétiques $M_1$, $M_2$, $M_3$ (ou $M_{10}$, $M_{20}$, $M_{30}$) afin d'éviter toute saturation du matériau magnétique constituant la pièce polaire $A_1$. Une bobine magnétique $b_1$ annulaire est disposée sur la pièce polaire $A_1$. En vis-à-vis de la pièce polaire $A_1$ est placée une pièce polaire $A_2$, identique, associée à la bobine magnétique annulaire $b_2$ identique à $b_1$ (figure 8).

En fonctionnement, dans les exemples représentés en figures 1 et 3 du dispositif suivant l'invention, les différentes trajectoires des particules convergent dans le plan H horizontal en un foyer $F_H$ situé dans la face S de sortie du troisième secteur magnétique $M_3$ (figure 9) tandis que dans le plan vertical V, l'ensemble du dispositif de déviation se comporte comme un espace de glissement. Si l'on souhaite conserver un dispositif de déviation magnétique stigmatique, c'est-à-dire permettant de former une image ponctuelle d'un point objet pour les particules situées hors de l'axe du faisceau $f_i$ incident, il faut compenser les divergences du faisceau à la fois dans le plan V vertical et dans le plan H horizontal. Pour cela, il suffit que la trajectoire moyenne du faisceau $f_i$ incident fasse avec la face d'entrée E du dispositif de déviation magnétique un angle un peu différent de $\pi/2$ (figure 10).

La figure 9 montre les effets de lentille obtenus avec un dispositif de déviation magnétique dont les faces d'entrée E et de sortie S sont orthogonales à la trajectoire moyenne du faisceau de particules.

La figure 11 représente l'action des lentilles magnétiques formées par le dispositif de déviation magnétique suivant l'invention, montré en figure 10, lorsque ce dispositif de déviation présente au faisceau une face d'entrée E faisant un angle un peu différent de $\pi/2$ avec la trajectoire moyenne de ce faisceau $f_i$ incident. Dans ce cas, le faisceau $f_i$ est soumis à la fois à une focalisation dans le plan H horizontal et dans le plan V vertical, cette double focalisation étant située à une distance 1 de la face S de sortie du dispositif de déviation, cette distance 1 correspondant par exemple à la distance séparant la face S de sortie du dispositif de déviation et une cible Q destinée à être bombardée par un faisceau sensiblement ponctuel.

Les exemples donnés ne sont pas limitatifs. En particulier, la réalisation du secteur magnétique intermédiaire $M_2$ (ou $M_{20}$) peut être différente des exemples présentés. Il peut en particulier former un élément séparé qui sera accolé aux secteurs d'extrémité $M_1$, $M_3$ (ou $M_{10}$, $M_{30}$).

Le dispositif de déviation magnétique suivant l'invention présente plusieurs avantages. Il est compact et simple à réaliser. De plus, il présente une grande bande passante. Il peut être avantageusement utilisé dans les appareils de radiothérapie, supprimant tout réglage du champ magnétique pour un large domaine d'énergie des particules.

## Revendications

1. Dispositif de déviation magnétique stigmatique et achromatique d'un faisceau de particules chargées, accélérées, comprenant au moins un électro-aimant muni de pièces polaires délimitant des entrefers formant des secteurs magnétiques disposés à la suite les uns des autres et dans lesquels sont créés des champs magnétiques de même sens et de valeurs déterminées, de façon à obtenir, dans ce dispositif, des trajectoires de particules ayant la forme de boucles dont les longueurs sont fonction du moment de quantité de mouvement des particules, l'ensemble de ces secteurs magnétiques ayant un plan de symétrie perpendiculaire au plan de la trajectoire moyenne du faisceau de particules et coupant ce plan suivant un axe XX de symétrie du dispositif de déviation, caractérisé en ce qu'il comporte un premier, un second et un troisième secteurs magnétiques accolés, en ce que ces secteurs présentent successivement au faisceau de particules une face d'entrée (E) plane, une première face galbée ($F_1$) commune aux premier et second secteurs accolés, une seconde face galbée ($F_2$) commune aux second et troisième secteurs accolés et une face de sortie (S) plane, les faces d'entrée (E) et de sortie (S) faisant entre elles un angle $2\alpha$, en ce que les différentes trajectoires des particules sont orthogonales aux faces galbées ($F_1$ et $F_2$) et à l'axe XX de symétrie, et en ce que les valeurs des inductions magnétiques créées dans les premier et troisième secteurs magnétiques sont égales à $KB_0$, $B_0$ étant la valeur de l'induction magnétique magnétique dans le second secteur magnétique et K un coefficient numérique inférieur à 1.

2. Dispositif de déviation magnétique suivant la revendication 1, caractérisé en ce que le rayon de courbure $r_1$ des trajectoires des particules dans les premier et troisième secteurs magnétiques et le rayon de courbure $r_2$ des trajectoires dans le second secteur magnétique intermédiaire sont liés par la relation :

$$\frac{r_2}{r_1} = K = 1 + \frac{\left(\dfrac{b}{R}\,\mathrm{tg}\,\dfrac{\theta}{2} - 1\right)\mathrm{tg}\,\alpha}{\cos\theta\,\mathrm{tg}\,\alpha + \sin\theta}$$

$r_1$ et $r_2$ étant fonction, pour des valeurs d'induction magnétique déterminées dans les différents secteurs magnétiques, des moments de quantité de mouvement de ces particules, b étant la distance séparant la trajectoire moyenne incidente du point I intersection de la face d'entrée (E) avec l'axe de symétrie XX du dispositif de déviation magnétique, $\theta$ étant l'angle total de déviation des particules dans les premier et troisième secteurs magnétiques, cet angle θ étant fonction du moment de quantité de mouvement de ses particules, pour des valeurs déterminées de l'induction magnétique, R étant le rayon de courbure des faces galbées ($F_1$ et $F_2$) respectivement communes aux premier et deuxième secteurs magnétiques, et aux deuxième et troisième secteurs magnétiques, et en ce que l'angle 2 φ de déviation des particules dans le deuxième secteur magnétique est égal à $2[\pi - (\alpha + \theta)]$.

3. Dispositif de déviation magnétique suivant la revendication 2, caractérisé en ce qu'il comporte une paire de pièces polaires de forme et de dimensions telles qu'elle délimite trois secteurs magnétiques ($M_1$, $M_2$, $M_3$) successifs, accolés, dans lesquels sont créées respectivement des inductions magnétiques de valeurs $B_0/2$, $B_0$, $B_0/2$, le rapport $K = r_2/r_1$ étant égal à 0,5, l'angle 2 α égal à $\pi/2$, et le rayon de courbure R des faces intermédiaires ($F_1$, $F_2$) égal à 2 b.

4. Dispositif de déviation magnétique suivant la revendication 3, caractérisé en ce que chacune des pièces polaires est constituée d'un premier élément $a_1$ en matériau magnétique sur lequel est fixé un second élément c, placé de façon à diminuer l'entrefer des pièces polaires et ayant la forme du secteur magnétique intermédiaire.

5. Appareil d'irradiation utilisant un faisceau de particules chargées accélérées, caractérisé en ce qu'il comporte un dispositif de déviation magnétique suivant l'une quelconque des revendications 1 à 4.

## Claims

1. Device for magnetic stigmatic and achromatic deflection of a bundle or beam of charged accelerated particles, comprising at least one electromagnet provided with pole pieces which define iron gaps forming magnetic sectors disposed in succession in which magnetic fields are produced with the same sense and predetermined values so that in said device particle trajectories are obtained having the form of loops whose lengths are a function of the momentum of the particle movement, the whole of said magnetic sectors having a plane of symmetry perpendicular to the plane of the mean trajectory of the particle beam and intersecting said plane along an axis XX of symmetry of the deflection device, characterized in that it comprises a first, a second and a third magnetic sector which adjoin each other, in that said sectors present to the particle beam successively a planar entry face (E), a first curved face ($F_1$) common to the first and second adjoined sectors, a second curved face ($F_2$) common to the second and third adjoined sectors and a planar exit face (S), the entry face (E) and the exit face (S) enclosing between them an angle 2 α, in that the different particle trajectories are perpendicular to the curved faces ($F_1$ and $F_2$) and to the axis XX of symmetry, and in that the values of the magnetic inductions produced in the first and third magnetic sectors are equal to $KB_0$, $B_0$ being the value of the magnetic induction in the second magnetic sector and K a numerical coefficient less than 1.

2. Device for magnetic deflection according to claim 1, characterized in that the radius of curvature $r_1$ of the particle trajectories in the first and third magnetic sectors and the radius of $r_2$ of the trajectories in the second intermediate magnetic sector are related by the relationship :

$$\frac{r_2}{r_1} = K = 1 + \frac{\left(\dfrac{b}{R} \, \text{tg} \, \dfrac{\theta}{2} - 1\right) \text{tg} \, \alpha}{\cos \theta \, \text{tg} \, \alpha + \sin \theta}$$

$r_1$ and $r_2$ being dependent for predetermined magnetic induction values in the different magnetic sectors on the momentums of said particles, b being the distance separating the mean incidence trajectory from the intersection point I of the entry face (E) with the axis XX of symmetry of the magnetic deflection device, θ being the total deflection angle of the particles in the first and third magnetic sectors, said angle θ depending on the momentum of the particles for predetermined values of the magnetic induction, R being the radius of curvature of the curved faces ($F_1$ and $F_2$) respectively common to the first and second magnetic sectors and to the second and third magnetic sectors, and in that the deflection angle 2 φ of the particles in the second magnetic sector is equal to $2 \pi - (\alpha + \theta)$.

3. Device for magnetic deflection according to claim 2, characterized in that it comprises a pair of pole pieces of form and dimensions such that they define three successive adjoined magnetic sectors ($M_1$, $M_2$, $M_3$) in which are produced respectively magnetic inductions of the value $B_0/2$, $B_0$ and $B_0/2$, the ratio $K = r_2/r_1$ being equal to 0.5, the angle 2 α equal to $\pi/2$ and the radius of curvature R of the intermediate faces ($F_1$, $F_2$) being equal to 2 b.

4. Device for magnetic deflection according to claim 3, characterized in that each pole piece is formed by a first element $a_1$ of magnetic material to which a second element c is secured located so as to diminish the iron gap of the pole pieces and having the form of the intermediate magnetic sector.

5. Irradiation apparatus employing a beam of charged accelerated particles, characterized in that it comprises a magnetic deflection device according to any one of claims 1 to 4.

## Ansprüche

1. Vorrichtung zur magnetischen stigmatischen und achromatischen Ablenkung eines Bündels geladener, beschleunigter Teilchen, mit wenigstens einem Elektromagneten, der mit Polstücken versehen ist, die Eisenspalte begrenzen, welche magnetische Sektoren bilden, die aufeinanderfolgend angeordnet sind und in denen Magnetfelder mit demselben Sinn und bestimmten Werten erzeugt werden, so daß in dieser Vorrichtung Teilchenbahnen erhalten werden, welche die Form von Schleifen aufweisen, deren Längen vom Impulsmoment der Teilchenbewegung abhängt, wobei die Gesamtheit dieser magnetischen Sektoren eine Symmetrieebene aufweist, die senkrecht zur Ebene der mittleren Bahn des Teilchenbündels ist und diese Ebene längs einer Symmetrachse XX der Ablenkvorrichtung schneidet, dadurch gekennzeichnet, daß sie einen ersten, einen zweiten und einen dritten Magnetsektor umfaßt, die aneinandergefügt sind, daß diese Sektoren dem Teilchenbündel nacheinander eine ebene Eintrittsfläche (E), eine erste gewölbte Fläche ($F_1$), die dem ersten und dem zweiten der aneinandergefügten Sektoren gemeinsam ist, eine zweite gewölbte Fläche ($F_2$), die dem zweiten und dritten der aneinandergefügten Sektoren gemeinsam ist, sowie eine ebene Austrittsfläche (S) darbieten, wobei die Eintrittsfläche (E) und die Austrittsfläche (S) untereinander einen Winkel von $2\alpha$ bilden, daß die verschiedenen Teilchenbahnen senkrecht zu den gewölbten Flächen ($F_1$ und $F_2$) und zu der Symmetrachse XX sind, und daß die Werte der magnetischen Induktion, die in dem ersten und dritten magnetischen Sektor erzeugt werden, gleich $KB_0$ sind, worin $B_0$ der Wert der magnetischen Induktion in dem zweiten magnetischen Sektor und K ein Zahlenkoeffizient ist, der kleiner als 1 ist.

2. Vorrichtung zur magnetischen Ablenkung nach Anspruch 1, dadurch gekennzeichnet, daß der Krümmungsradius $r_1$ der Teilchenbahnen in dem ersten und in dem dritten magnetischen Sektor sowie der Krümmungsradius $r_2$ der Bahnen in dem zweiten, dazwischen angeordneten magnetischen Sektor durch folgende Beziehung verknüpft sind :

$$\frac{r_2}{r_1} = K = 1 + \frac{\left(\frac{b}{R}\,\mathrm{tg}\,\frac{\theta}{2} - 1\right)\mathrm{tg}\,\alpha}{\cos\theta\,\mathrm{tg}\,\alpha + \sin\theta}$$

wobei $r_1$ und $r_2$ für bestimmte Werte der magnetischen Induktion in den verschiedenen magnetischen Sektoren von den Impulsmomenten dieser Teilchen abhängen, während b die Entfernung zwischen der mittleren Ankunftsbahn und dem Schnittpunkt I der Eintrittsfläche (E) mit der Symmetrieachse XX der magnetischen Ablenkvorrichtung ist sowie $\theta$ der Gesamtablenkwinkel der Teilchen in dem ersten und dritten magnetischen Sektor ist, wobei dieser Winkel $\theta$ von dem Impulsmoment der Teilchen für bestimmte Werte der magnetischen Induktion abhängt, und wobei R der Krümmungsradius der gewölbten Flächen ($F_1$ und $F_2$) ist, die jeweils dem ersten und zweiten der magnetischen Sektoren gemeinsam sind bzw. dem zweiten und dritten der magnetischen Sektoren gemeinsam sind, und daß der Ablenkwinkel $2\varphi$ der Teilchen in dem zweiten magnetischen Sektor gleich $2[\pi - (\alpha + \theta)]$ ist.

3. Vorrichtung zur magnetischen Ablenkung nach Anspruch 2, dadurch gekennzeichnet, daß sie zwei Polstücke aufweist, deren Form und Abmessungen derart sind, daß sie drei aufeinanderfolgende und aneinandergefügte magnetische Sektoren ($M_1$, $M_2$, $M_3$) bilden, in denen eine magnetische Induktion mit dem Wert $B_0/2$, $B_0$ bzw. $B_0/2$ erzeugt wird, wobei das Verhältnis $K = r_2/r_1$ gleich 0,5 ist, der Winkel $2\alpha$ gleich $\pi/2$ ist und der Krümmungsradius R der Zwischenflächen ($F_1$, $F_2$) gleich 2 b ist.

4. Vorrichtung zur magnetischen Ablenkung nach Anspruch 3, dadurch gekennzeichnet, daß jedes der Polstücke aus einem ersten Element $a_1$ aus Magnetmaterial gebildet ist, an dem ein zweites Element c befestigt ist, das eine solche Lage aufweist, daß der Eisenspalt der Polstücke verkleinert wird, und welches die Form eines magnetischen Zwischensektors aufweist.

5. Bestrahlungsgerät, in dem ein Bündel geladener und beschleunigter Teilchen zur Anwendung gelangt, dadurch gekennzeichnet, daß es eine magnetische Ablenkvorrichtung nach einem der Ansprüche 1 bis 4 enthält.

Fig.1

Fig.2

Fig.3

Fig.4

$$K = \frac{r_2}{r_1}$$

$$\frac{b}{R} = 0,5$$

$$\alpha = 45°$$

FIG.5

$$K = \frac{r_2}{r_1}$$

$$\frac{b}{R} = 0,63$$

$$\alpha = 60°$$

| 1,9E_o | 1,73E_o | 1,43E_o | 1,19E_o | E_o | 0,9E_o | 0,84E_o |

FIG.6

3

Fig. 7

Fig. 8

4

E                   S

$\underline{H}$

L

$\underline{V}$

$F_H$

Fig. 9

$F_2$

$\underline{M}_3$      $\underline{M}_2$

$F_1$

S    I

E   $\underline{M}_1$

$\alpha + \beta$

$t_1$

$f_i$    $f_e$    $\beta$

Fig. 10

$L_1$      $L_2$              $\ell$

$\underline{H}$

$\underline{V}$                            $F_H$

$F_V$

E                    S

Fig. 11

5